# EUROPEAN PATENT APPLICATION

(11) **EP 4 410 301 A1**
(43) Date of publication of application: **07.08.2024**
(21) Application number: 21958636.9
(22) Date of filing: 28.09.2021
(51) Int. Cl.: A61K 38/13, A61K 47/54, A61P 3/10, A61P 9/10, A61P 35/00

(54) **EXTRACELLULAR CYCLOPHILIN INHIBITOR AND USE THEREOF**

(71) Applicant: Peking University Shenzhen Graduate School, Shenzhen, Guangdong 518055 (CN)
(72) Inventor: QUAN, Junmin, Shenzhen, Guangdong 518000 (CN); YANG, Zhen, Shenzhen, Guangdong 518000 (CN); LIU, Siyu, Shenzhen, Guangdong 518000 (CN); ZHANG, Qingzhou, Shenzhen, Guangdong 518000 (CN); HU, Minqiang, Shenzhen, Guangdong 518000 (CN); LI, Fengxia, Shenzhen, Guangdong 518000 (CN); FU, Jiamiao, Shenzhen, Guangdong 518000 (CN); ZHU, Zhendong, Shenzhen, Guangdong 518000 (CN); LI, Qinkai, Shenzhen, Guangdong 518000 (CN)
(74) Representative: dompatent von Kreisler Selting Werner - Partnerschaft von Patent- und Rechtsanwälten mbB
(86) International application number: PCT/CN2021/121201
(87) International publication number: WO 2023/050037

(57) **Abstract**

The present invention belongs to the technical field of biomedicine, and specifically relates to an extracellular cyclophilin inhibitor and the use thereof, wherein the structural general formula of the extracellular cyclophilin inhibitor of the present invention is as shown in formula (I). According to the present invention, a group capable of reacting with sulfhydryl of cysteine at a specific site on blood albumin is extracted by means of a side chain of a new cyclosporin derivative, and a drug conjugate is rapidly formed after same enters the body, which can effectively restrict the drug to the extracellular area and form targeted inhibition of extracellular cyclophilin, thereby achieving the aim of treating diseases associated therewith.

## Description

### FIELD

The present invention relates to the field of biomedicine, and in particular to an extracellular cyclophilininhibitor and use thereof.

### BACKGROUND

Cyclophilins (CyPs), a multifunctional protein family with high conservation and widely distributed in nature, have been identified as the major intracellular binding protein of the immunosuppressive drug Cyclosporin A (CsA). Cyclophilins have the activity of peptidyl-prolyl cis-trans isomerase (PPIase) and chaperone function and can assist the correct folding of intracellular proteins, participate in immune suppression, mediate inflammatory response, and participate in oxidative stress response and other biological functions. Cyclophilins are mainly localized in the cytoplasm and play important roles in many life processes. In oxidative stress state or inflammatory environment, Cyclophilin A (CypA) can be secreted out of cells via vesicle transport to form extracellular CypA (eCypA), which then binds to membrane receptors to initiate signal transduction reactions in target cells, chemotaxis of immune cells such as neutrophils, eosinophils, and T cells to mediate immune response and inflammatory response, which are closely related to a variety of inflammation-related diseases. Different from intracellular and extracellular distribution, CypA plays different roles.

Extracellular cyclophilins are thought to play a critical role in a variety of serious diseases. In chronic inflammatory conditions, monocytes, vascular smooth muscle cells, and endothelial cells, when stimulated by ROS and inflammation, secrete large amounts of extracellular cyclophilins out of the cell via vesicles. Cyclophilins secreted into the extracellular matrix can also interact with membrane proteins, stimulate the activation of ERK1/2, AKT, JAK, NF-KB, and JNK, further amplify ROS and inflammation, and induce monocytes and macrophages to secrete IL-1β, IL-6, and IL-8. It can activate matrix metalloproteinases MMP-2 and MMP-9, and promote proliferation and migration of vascular smooth muscle cells. The role of extracellular cyclophilins in these pathways mediates tumor proliferation and migration, insulin resistance, cardiovascular disease, neurodegeneration, and many other disease processes; extracellular cyclophilins are upregulated in many of the above chronic inflammatory conditions and play a key role in the development of these diseases, such as cancer, rheumatoid arthritis, airway inflammation, diabetes, hypertension, cardiovascular diseases, lupus, skin diseases, xerophthalmia, intestinal diseases, abnormal lipid metabolism, and aging. Inhibitors of cyclophilins have been reported to have a wide therapeutic range, such as anti-infection, asthma, pneumonia, cardiovascular diseases, diabetes, promotion of hair growth, suppression of immune responses, arthritis, dermatitis, psoriasis, multiple sclerosis, tumors, and Alzheimer's disease. Inhibitors of cyclophilins are therefore an important class of drugs.

Cyclosporine A (CsA), as the first natural cyclophilin inhibitor, inhibits the dephosphorylation of NFAT by calcineurin by forming a complex with intracellular CypA, thus inhibiting the activation of T cells, and playing an immunosuppressive role. It has been widely used in organ transplantation and autoimmune diseases. CsA inhibits both extracellular and intracellular CyP function and is therefore also used in a variety of inflammatory diseases. However, in obtaining extracellular effects specific for extracellular cyclophilins, higher doses are often required to compensate for the "loss" of the molecule into the cell. However, due to many important functions of intracellular cyclophilins, the toxicity of CsA at high doses, especially islet damage and hepatorenal toxicity, greatly limits its development. Thus, if compounds that inhibit extracellular cyclophilins could be developed without entering the cell while producing the desired therapeutic effect, it would be possible to avoid these undesirable side effects.

Existing technologies can effectively reduce inflammation such as respiratory and cardiovascular diseases by targeting secreted extracellular cyclophilins. By adding carboxybenzimidazole-modified MM284 to CsA, the hydrophilicity of the molecule can be improved without cell penetration, limiting its effect to the extracellular part of cyclophilins, effectively inhibiting the migration of immune cells, thus reducing inflammation. A similar molecule, MM218, prevents CsA from entering cells by adding a rhodamine side chain, thereby reducing lung inflammation, and improving asthma, but may cause some adverse reactions due to its ability to interact with multidrug-resistant transport systems. In addition, although this kind of chemically modified CsA can achieve no entry into cells with the help of negatively charged groups, there is also an obvious problem that its accumulation in the body is also reduced due to the improvement of hydrophilicity, and it is quickly eliminated by the kidney, and the metabolism is fast, and it is difficult to play a long-term role.

### SUMMARY

An object of the present invention is to provide an extracellular cyclophilin inhibitor, 4MCsA.

A further object of the present invention is to provide the use of the above compound 4MCsA.

The extracellular cyclophilin inhibitor according to a specific embodiment of the present invention has the structure shown in Formula (I): 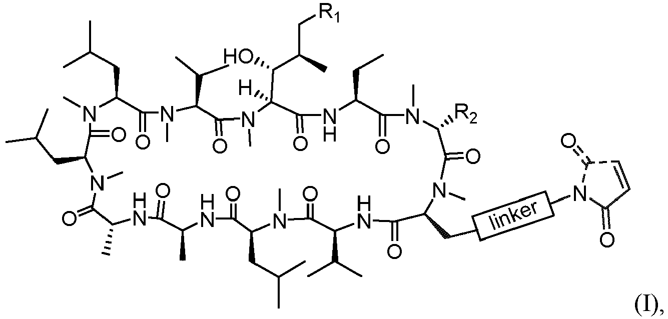 wherein, R₁ is -CH=CHR₁' or -CH₂CH₂R₁'; R₁' is selected from alkyl, carboxyl, acetamino, or phenyl;
R₂ is H, SR₂', CH₂SR₂', or CH₂OR₂'; R₂' is selected from alkyl, carboxyl, hydroxy, acetamino, or phenyl; and
the linker is selected from -(CH₂)*ₓ*-NH(C=O)-(CH₂)*_{y}*- or -(CH₂)*ₓ*-NH(C=O)-(CH₂CH₂O)*_{y}*-; wherein, *x* is an integer of 1-4, and *y* is an integer of 1-6.

The extracellular cyclophilin inhibitor according to a specific embodiment of the present invention, R₁' is selected from -CH₃, -(CH₂)*ₙ*-COOH, -(CH₂)*ₙ*-NH(C=O)CH₃, -phenyl, or cycloalkyl; wherein, *n* is an integer of 1-6, *i.e. n* is 1, 2, 3, 4, 5, or 6;
the phenyl is substituted, one or more, identically or differently, by the following groups: - COOCH₃ or -CH₂NH(C=O)CH₃.

The cycloalkyl is preferably cyclopropyl.

The extracellular cyclophilin inhibitor according to a specific embodiment of the present invention, R₂' is selected from -CH₃, -(CH₂)*ₙ*-COOH, -(CH₂)*ₙ*-OH, -(CH₂)*ₙ*-NH(C=O)CH₃, - phenyl, or cycloalkyl, *n* is an integer of 1-6; the phenyl is substituted, one or more, identically or differently, by the following groups: -COOCH₃ or -CH₂NH(C=O)CH₃.

In the present invention, an "*alkyl*", *i.e.* a saturated hydrocarbon group, is a hydrocarbon group formed by removing one hydrogen atom from the molecule of an alkane and is a kind of chain organic group containing only carbon and hydrogen atoms. Suitable alkyls in the present invention include but are not limited to, methyl CH₃-, ethyl CH₃CH₂-, CH₃CH₂CH₂-, and the like.

The term "*cycloalkyl*" in the present invention includes saturated monocyclic, bicyclic, tricyclic, or polycyclic hydrocarbon groups having 3 to 12 carbon atoms, any ring atom capable of substitution may be substituted by a substituent. Examples of cycloalkyl include but are not limited to, cyclopropyl, cyclobutyl, and cyclohexyl.

The extracellular cyclophilin inhibitor according to a specific embodiment of the present invention, R₁ is -CH=CHCH₃, R₂ is H, and linker is selected from -(CH₂)*ₓ*-NH(C=O)-(CH₂)*_{y}*- or - (CH₂)*ₓ*-NH(C=O)-(CH₂CH₂O)*_{y}*-; *x* is an integer of 1-4, and y is an integer of 1-6.

Preferably, R₁ is -CH=CHCH₃, R₂ is H, and linker is -(CH₂)*ₓ*-NH(C=O)-(CH₂)*_{y}*-; *x* is 2 or 3, and y is 3, 4, or 5.

More preferably, R₁ is -CH=CHCH₃, R₂ is H, and linker is -(CH₂)₃-NH(C=O)-(CH₂)₅-.

The present invention also provides a pharmaceutical composition of extracellular cyclophilin inhibitor and a non-toxic pharmaceutically acceptable salt thereof as active ingredients.

The term *"pharmaceutically acceptable salt"* refers to a salt that retain the biological effectiveness and properties of the compounds of this invention and which are not biologically or otherwise undesirable. In many instances, the compounds of the present invention utilize the presence of amino and/or carboxyl groups or similar groups to form an acid and/or base salt. A pharmaceutically acceptable acid addition salt may be prepared from inorganic or organic acids, while a pharmaceutically acceptable base addition salt may be prepared from inorganic or organic bases. For a review of pharmaceutically acceptable salt, *see* Berge et al. ((1977) J. Pharm. Sd, Vol. 66, 1). *"Non-toxic pharmaceutically acceptable salt*" refers to a non-toxic salt formed with non-toxic pharmaceutically acceptable inorganic or organic acids or inorganic or organic bases. For example, the above-mentioned salt include those derived from inorganic acids such as hydrochloric, hydrobromic, sulfuric, sulfamic, phosphoric, nitric and the like, as well as from organic acids such as acetic, propionic, succinic, glycolic, stearic, lactic, malic, tartaric, citric, ascorbic, pamoic, maleic, hydroxymaleic, phenylacetic, glutamic, benzoic, salicylic, sulfanilic, fumaric, methanesulfonic, toluenesulfonic acids, and the like.

The compound of formula (I) of the present invention can be administered alone, preferably they are provided as a pharmaceutical composition. The above pharmaceutical compositions useful according to the present invention, whether for veterinary use or for human use, include at least one compound of formula (I) as defined above, together with one or more pharmaceutically acceptable carriers therefor and optionally other therapeutic ingredients.

In certain preferred embodiments, the active ingredients required for combination therapy may be combined in a single pharmaceutical composition for simultaneous administration.

As used herein, the term *"pharmaceutically acceptable"* and grammatical variations thereof when referring to compositions, carriers, diluents, and agents, are used interchangeably and mean that the substance may be administered to a mammal without adverse physiological effects such as nausea, dizziness, gastric upset, and the like.

Methods of preparing pharmaceutical compositions in which an active ingredient is dissolved or dispersed are known in the art and need not be limited based on formulation. Typically, such compositions are prepared as injections, either as liquid solutions or suspensions; however, solid forms suitable for solution or suspension in a liquid before use may also be prepared. The formulation may also be emulsified. In particular, the pharmaceutical compositions described above may be formulated as solid dosage forms, such as capsules, tablets, pills, powders, dragées, or granules.

The choice of excipient and the amount of active ingredient in the excipient are generally determined by the solubility and chemical properties of the active compound, the specific mode of administration, and the requirements observed in pharmaceutical practice. For example, excipients such as lactose, sodium citrate, calcium carbonate, and dicalcium phosphate and disintegrants such as starch, alginic acid, and certain complex silicates, in combination with lubricants such as magnesium stearate, sodium lauryl sulfate, and talc may be used to prepare tablets. For the preparation of capsules, it is advantageous to use lactose and high molecular weight polyethylene glycols. When an aqueous suspension is employed, it may contain emulsifying agents or suspension-promoting agents. Diluents such as sucrose, ethanol, polyethylene glycol, propylene glycol, glycerol, and chloroform or mixtures thereof may also be used.

The pharmaceutical compositions described above may be administered to humans and animals in suitable dosage forms for local or systemic administration, including oral, rectal, nasal, buccal, ocular, sublingual, transdermal, rectal, topical, vaginal, parenteral (including subcutaneous, intraarterial, intramuscular, intravenous, intradermal, intrathecal, and epidural), intracisternal and intraperitoneal administration. It will be appreciated that the preferred route may vary with, for example, the condition of the recipient.

The formulations may be prepared in unit dosage form by any of the methods well known in the art of pharmacy. Such methods include the step of bringing into association the active ingredient with the carrier which constitutes one or more accessory ingredients. In general, the formulations are prepared by uniformly and intimately bringing into association the active ingredient with liquid carriers or finely divided solid carriers or both, and then, if necessary, shaping the product. A total daily dose of a compound of the present invention administered to a subject in single or multiple doses.

The present invention also provides the use of an extracellular cyclophilin inhibitor as described above in preparing a drug for the prevention or treatment of a disease mediated by cyclophilins; the disease mediated by cyclophilins includes, but is not limited to, a) viral infections, b) metabolic diseases, c) acute and chronic inflammatory diseases, d) cancer, e) neurodegenerative diseases, f) degenerative muscle diseases, g) cardiovascular diseases, h) obesity, and i) diabetes. Beneficial effects of the present invention:

The present invention, based on the active pocket of cyclophilins and the known structureactivity relationship of CsA, results in a novel 4MCsA compound, which is a potent inhibitor of extracellular cyclophilins by limiting the effect of the compound extracellularly and simultaneously increasing the metabolic time of the compound and improving the specificity after highly efficient reaction with albumin. The compound, or a corresponding pharmaceutically acceptable salt form thereof, binds to extracellular cyclophilins and thereby inhibits the amplification of chronic inflammation and exhibits good therapeutic effects in a variety of chronic inflammatory diseases, such as tumors, type II diabetes, and atherosclerosis.

### BRIEF DESCRIPTION OF THE DRAWINGS

To explain the examples of the present disclosure or the technical solutions in the prior art more clearly, a brief introduction will be made to the accompanying drawings used in the examples or the description of the prior art. It is obvious that the drawings in the description below are only some examples of the present disclosure, and those ordinarily skilled in the art can obtain other drawings according to these drawings without creative work.
Fig. 1 shows a mass spectrum of a 4MCsA modified albumin, wherein, the top is BSA and the bottom is BSA-4MCsA conjugate;
Fig. 2 shows results of the identification of the Cysteine modification site on BSA by 4MCsA, the top shows the 2+ ion of the corresponding peptide fragment of a BSA sample, and the bottom shows the 3+ ion of the corresponding peptide fragment of a BSA-4MCsA;
Fig. 3 shows EIC plots of CsA, 4MCsA, and thiophenol blocked 4MCsA (Ar-S-4MCsA), I.F. represents the intracellular extract fraction, E.S. is the curve of the standard, the area under the curve is used for quantitative calculation of intracellular concentration, the shaded part of the plot is the area under the I.F. curve;
Fig. 4 is a competitive flow cytometry image of CsA-FITC fluorescently labeled Jurkat cells;
Fig. 5 shows a demonstration of the in vitro activity of 4MCsA molecules, wherein, A is a competitive fluorescence polarization assay for the binding ability of CsA (•), 4MCsA (▪) and BSA-4MCsA (▲) to CypA; B is CsA (•), 4MCsA (▪) and BSA-4MCsA (▲) inhibits cis-trans isomerase activity of CypA in a dose-dependent manner; C is the corresponding *Kᵢ* and IC50 for each inhibitor;
Fig. 6 shows that the 4MCsA molecule has no immunosuppressive activity; wherein, A is the inhibition of dephosphorylation of NFAT 1 induced by 25 nM PMA and 1 mg/mL ionomycin in Jurkat cells by Western Blot detection molecule (band with lower molecular weight), and B is the secretion of IL-2 after activation of Jurkat cells by ELISA detection;
Fig. 7 shows that 4MCsA inhibits inflammation and monocyte migration stimulated by eCypA; wherein, A is the IL-8 expression of CypA-induced THP-1 cells, and the mRNA level of IL-8 was significantly increased in THP-1 cells treated with 100 nM CypA for 12 h, while 4MCsA and CsA could inhibit this effect at the same time; B is the CypA-induced THP-1 cell migration coefficient; C is a representation of THP-1 cells stained with crystal violet in cell migration assays;
Fig. 8 shows the cytotoxic effect of CsA and 4MCsA on human kidney cells HEK293;
Fig. 9 shows disease status and serum CypA contents following treatment with CypA inhibitors in a mouse model of T2DM;
Fig. 10 shows glycemic control of 4MCsA and CSA administered by tail vein injection in DB and HFD mice, wherein, a is the fasting blood glucose of DB mice, b is fasting blood glucose of C57 mice; c is the body weight of DB mice after administration; d is the body weight of HFD and DB mice relative to the corresponding C57 control; e is fasting blood glucose of HFD mice; f is the control of HbAlc of HFD by 4MCsA and CsA 5 mg/kg/week;
Fig. 11 shows an effect of different drugs and doses on GTT in DB mice;
Fig. 12 shows an effect of 4MCsA and CsA in HFD mice and their different time intervals after administration on GTT;
Fig. 13 shows an effect of CsA and 4MCsA on insulin, wherein, a is the basal level of DB mouse insulin after administration and b is the DB mouse insulin secretion in response to glucose stimulation after administration;
Fig. 14 shows an effect of CsA and 4MCsA on mouse ITT experiments, wherein, a and c are ITT after administration to DB mice, b and d are ITT responses in C57 mice; e and f are ITT after administration in HFD mice;
Fig. 15 is graphs showing the levels of inflammatory factors in the blood of DB mice after administration; a is the level of IL-1β; b is the level of CRP; c is the level of TNF-α; d is the level of IL-6;
Fig. 16 shows results of hepatorenal toxicity evaluation of DB mice after administration, wherein, a is the level of aspartate aminotransferase AST; b is the level of alanine aminotransferase ALT; c is the level of urea; d is the level of creatinine;
Fig. 17 shows a change of tumor volume in tumor model mice;
Fig. 18 shows an effect of 4MCsA on CD8⁺T cells in the tumor microenvironment;
Fig. 19 shows an effect of 4MCsA on the extent of aortic lesions in high-fat diet-induced atherosclerotic mice;
Fig. 20 shows an effect of 4MCsA on serum CRP contents in high-fat diet-induced atherosclerotic mice;
Fig. 21 shows an effect of 4MCsA on conventional blood lipid markers in serum of high-fat diet-induced atherosclerotic mice, wherein, a is the serum total cholesterol (TC) content; b is the serum triglyceride (TG) content; c is the content of low-density lipoprotein cholesterol (LDL-C); d is the content of high-density lipoprotein cholesterol (HDL-C); e is the ratio of total cholesterol to high-density lipoprotein cholesterol (TC/HDL-C); and
Fig. 22 shows an effect of chronic injection of 4MCsA on ALT, and AST contents in serum of high-fat diet-induced atherosclerotic mice.

### DETAILED DESCRIPTION

To make the objects, aspects, and advantages of the present invention more apparent, a detailed description of the technical solutions of the present invention will be provided below. The described examples are not all but only part of examples of the present invention. Based on the examples of the present invention, all other examples obtained by a person of ordinary skill in the art without inventive effort fall within the scope of the present invention.

The present invention is exemplified by 4MCsA modified with 4-position modified maleimide, which can achieve the effect of limiting the molecule extracellularly by introducing a maleimide reactive group at 4-position of CsA to covalently react with BSA.

To selectively target eCypA, a previous modification (MM284) introduced a negatively charged group at the 1-double bond of cyclosporin. This position is easier to modify due to the presence of the 1-position double bond of cyclosporin, however, this position is also the site where cyclosporin binds to cyclophilins and is not suitable for introducing larger groups. Moreover, such CsA analogs are often rapidly cleared by the kidneys in the blood circulation, thereby preventing further clinical use. Cyclosporine binding to cyclophilins, in which the P4 position is located away from cyclophilins, is an ideal modification site. However, the modification of this site is difficult and requires a complex synthetic route. Cyclosporine, as a complex natural product, has a molecular weight of more than 1000 Da. Thus, coupling proteins with cyclosporin derivatives has not been used for pharmaceutical applications.

The present invention, to obtain an active product of position 4 of cyclosporin coupled with albumin, introduces Michael acceptor derivative molecules 3 and 8 at the cyclosporine position 4, and use the same for cyclosporin-albumin coupling and activity verification.

The synthetic process of 4MCsA of the present invention uses only two-step column chromatography for purification, which greatly reduces the operation difficulty and greatly improves the yield.

The synthetic route of the reaction is as follows:

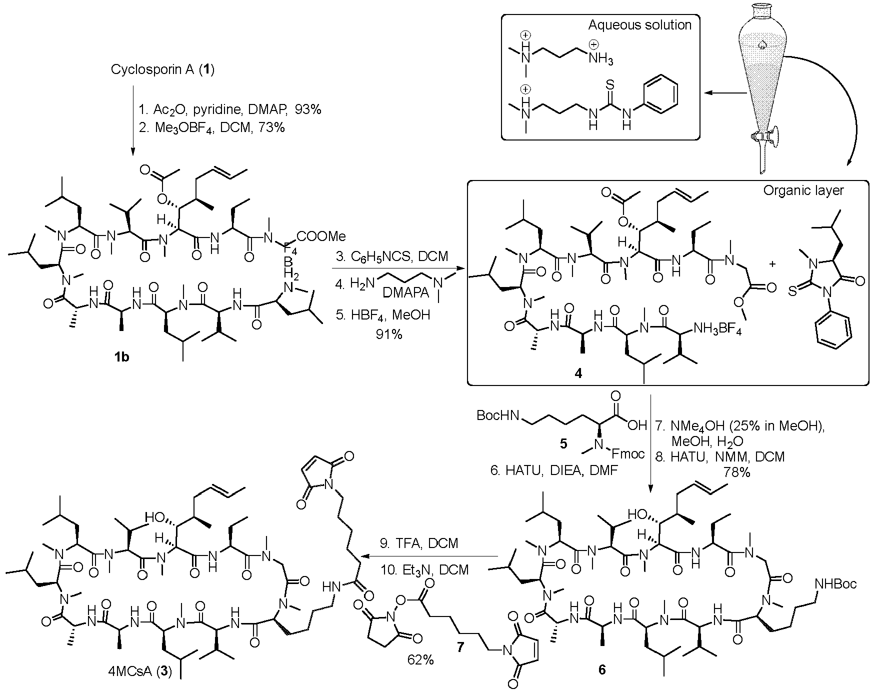

The synthetic scheme of the present invention includes two main steps:
The first is the synthesis of decapeptide **4** from CsA (**1**) with ring opening at positions P3 and P4 and removal of the amino acid at position P4. The key challenge in synthesizing **4** is the Edman degradation step, which in most cases results in a mixture of the undecapeptide (**1b**), the decapeptide (**4**), and the valine-deleted nonapeptide. The present invention uses an excess (2.0 to 5.0 equiv.) of PhNCS to react well with 1a and then captures unreacted PhNCS with DMAPA. In this way, excess DMAPA after Edman degradation can be washed away to obtain intermediate **4** in high purity.

The second is the coupling of decapeptide **4** with the amino acid Fmoc-*N*-Me-Lys (Boc)-OH (**5**), followed by removal of the Fmoc, acetyl group with NMe4OH and hydrolysis of the methyl ester, and finally, macrocyclization with HATU to give the cyclic peptide [MeLys(Boc)₄]CsA (**6**).

[MeLys(Boc)₄]CsA (**6**) is deprotected under trifluoroacetic acid (TFA) to give [MeLys₄]-CsA, which is then coupled with 6-maleimidocaproic acid-*N*-succinimidyl ester (7) to give 4MCsA (3).

Finally, starting from CsA (**1**) in 10 steps, and two-step column chromatography purification, 4MCsA (**3**) was synthesized in 30% overall yield.

### Example 1 Synthesis of linear decapeptides

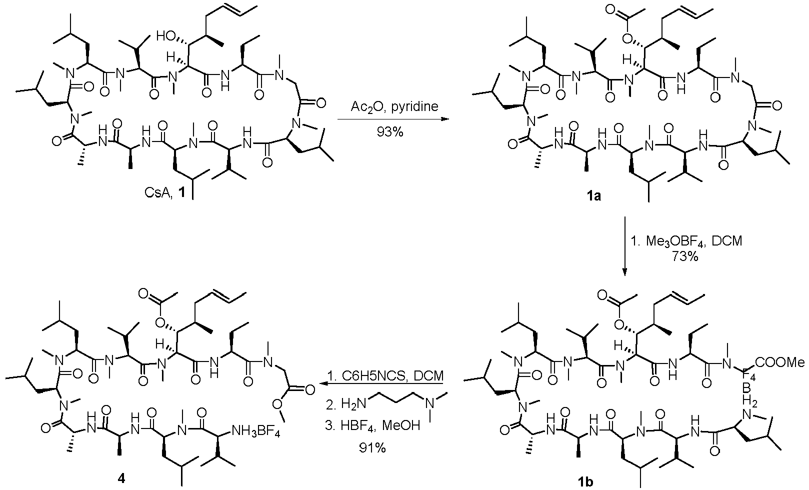

CsA (24.0 g, 20 mmol, 1.0 equiv.) was dissolved in a mixture of acetic anhydride (80 mL) and pyridine (80 mL). DMAP (0.8 g, 6.5 mmol, 0.33 equiv.) was added at 0°C and the reaction was stirred overnight. Upon complete consumption of CsA by LC/MS, the reaction was placed in an ice bath, and ice water (100 mL) was added dropwise as a white solid precipitated. A white solid was then isolated by filtration and the crude product was further dissolved in DMF (72 mL) and precipitated by the addition of water (72 mL). After washing, filtration, and drying, **1a** (23.2 g, 18.6 mmol, 93%) was obtained as a white solid. HRMS (ESI) m/z for C₆₄H₁₁₃N₁₁NaO₁₃⁺([M+Na]⁺) calculated: 1266.8412; found: 1266.8446.

**1a** (37.4 g, 30 mmol, 1.0 equiv.) was dissolved in DCM (300 mL) and trimethoxy tetrafluoroborate (13.3 g, 90 mmol, 3.0 equiv.) was added. After 20 hours of reaction, acetonitrile and water were added and reacted for another 3 hours. The organic phase was separated and washed with water (3 × 100 mL). Then, the reaction solution was concentrated and dried under a vacuum. The white crystalline powder was then crystallized in a mixture of 2-methyl tetrahydrofuran (100 mL) and methyl tert-butyl ether (50 mL) to form **1b** (29.9 g, 21.9 mmol, 73%). HRMS (ESI) m/z calculated for C₆₅H₁₁₈N₁₁O₁₄⁺([M+H]⁺c 1276.8854; found: 1276.8882.

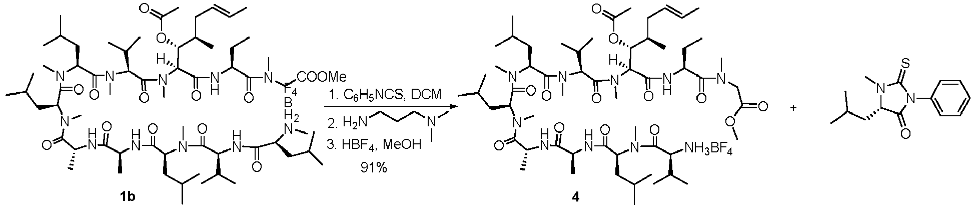

**1b** (13.7 g, 10.0 mmol) was dissolved in DCM (100 mL), DIPEA (6.6 mL, 40 mmol, 4.0 equiv.) and phenyl isothiocyanate (4.1 g, 30 mmol, 3.0 equiv.) were added, the reaction was stirred for 2.0 h, *N*,*N*-dimethyl-1,3-propanediamine (3.2 mL, 25 mmol, 2.5 equiv.) was added and stirring was continued for 60 min. Methanol (60 mL) and 50% aqueous fluoroboric acid (23 g, 130 mmol) were then added and the reaction was monitored by LCMS until complete. The reaction was diluted with DCM and washed with 1 N HCl and brine. The organic phases were combined, concentrated, and recrystallized from a mixed solution of 2-methyl tetrahydrofuran (50 mL) and methyl tert-butyl ether (25 mL) to give the product **4** as a white foam (11.2 g, 91%). HRMS (ESI) m/z for C₅₈H₁₀₅N₁₀O₁₃⁺([M+H]⁺) calculated: 1149.7857; found: 1149.7902.

### Example 2 Synthetic procedure from decapeptide to 4MCsA

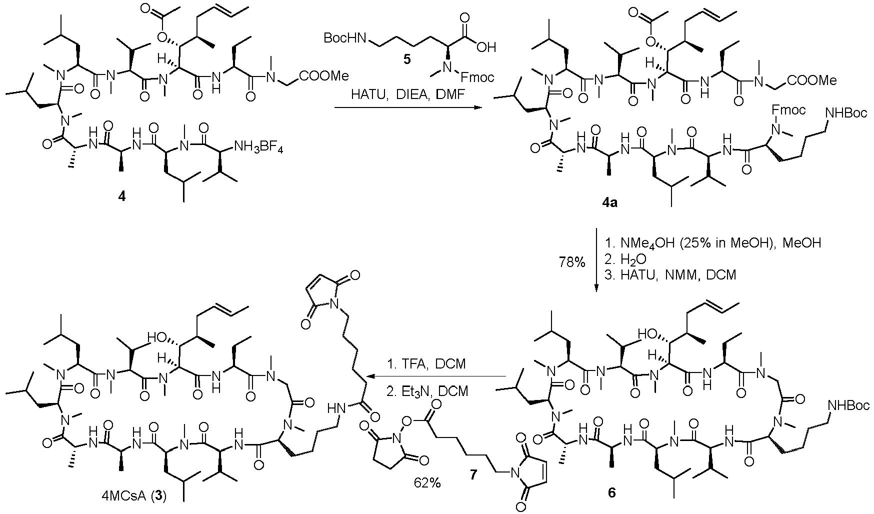

**4a**: Linear decapeptide **4** (1.20 g, 0.97 mmol, 1.0 equiv.) was dissolved in 10 mL of anhydrous DMF and the addition of amino acids Fmoc-N-Me-Lys (Boc)-OH (**5**) (468 mg, 0.97 mmol, 1.0 equiv.) and HATU (737mg, 1.94mmol, 2.0 equiv.) was continued with stirring. After the reaction was cooled to 0°C with an ice bath, DIPEA (0.68 mL, 3.88 mmol, 4.0 equiv.) was added and the solution was stirred at room temperature overnight. After completion of the reaction as detected by LCMS, the reaction mixture was quenched by the addition of 1 N HCl (10 mL) solution, and the resulting mixture was extracted with DCM (2×30 mL). The combined organic layers were washed with brine and dried over sodium sulfate. The extract was concentrated *in vacuo* and the residue was dissolved in 15 mL of methanol. Characterization data for **4a**: MS (ESI) m/z for C₈₃H₁₃₅N₁₂O₁₇⁺ [M+H]⁺ calculated: 1572.0, found: 1572.0.

**6**: A solution of **4a** in methanol was added 5 mL NMe₄OH (25% in methanol) and the reaction was stirred at room temperature. After LCMS detected removal of the acetyl and N-terminal Fmoc at position P1, 0.5 mL of water was added to the solution and stirred for 30 minutes to hydrolyze the methyl ester at position P3. After completion of the reaction, the reaction was quenched by adjusting pH to 5.0 by addition of 1 N HCl and the resulting mixture was extracted with DCM. The combined organic layers were washed with brine and dried over sodium sulfate. The solvent of the extract was concentrated *in vacuo* and redissolved in 200 mL of anhydrous DCM. HATU (1.32 g, 3.47 mmol, 3.85 equiv.) was then added to the stirred solution. After the HATU dissolved, the ice bath was cooled to 0°C, and N-methylmorpholine (0.76 mL, 6.94 mmol, 7.7 equiv.) was added dropwise. After completion of the reaction as detected by LCMS, the reaction mixture was washed with 1 N HCl, water, and brine. The organic layer was dried over sodium sulfate, concentrated in vacuo and the residue was purified by flash chromatography on silica gel (DCM: MeOH = 20: 1) to give **6** (992 mg, 78% yield) as a white solid.

Characterization data for **6**: ¹H NMR (500 MHz, CDCl₃) δ 7.88 (d, J=9.6 Hz, 1 H), 7.75 (d, J=7.3 Hz, 1 H), 7.43 (d, J=8.4 Hz, 1 H), 7.15 (d, J=7.8 Hz, 1 H), 3.49 (s, 3 H), 3.36 (s, 3 H), 3.22 (s, 3 H), 3.12 (s, 3 H), 3.07 (s, 3 H), 2.72 (s), 3 H), 2.69 (s, 3 H). HRMS(ESI)m/z: for C₆₇H₁₂₀N₁₂NaO₁₄⁺([M+Na]⁺) calculated: 1339.8939; found: 1339.8937.

4MCsA (**3**): **6** (89 mg, 66.4 mol, 1.0 equiv.) was dissolved in 2 mL DCM, then 650 L of TFA was added and stirred for 30 minutes. The solution was then concentrated and the residue was dissolved in DCM, washed with water, and saturated with NaHCO₃ and brine. The organic layer was dried over anhydrous sodium sulfate and concentrated in vacuo. The residue was dissolved in 1.0 mL anhydrous DCM, then N-succinimidyl-6-maleimidocaproate (7) (31 mg, 0.1 mmol, 1.5 equiv.) and Et₃N (28 µL, 0.2 mmol, 3.0 equiv.) were added. The solution was stirred overnight. The reaction mixture was then diluted with 10 mL DCM and washed with 1N HCl, water, and brine. The organic layer was dried over sodium sulfate, concentrated in vacuo and the residue was purified by HPLC to afford 4MCsA (3) (58 mg, 41.1 [µmol, 62% yield) as a white solid.

Characterization data for 4MCsA (3): HPLC purification method, gradient (70% MeOH 10 min, 70%-100% 10-25 min, 100% 25-35 min), Rt = 32.6 min. ¹H NMR(500 MHz, CDCl₃) δ 7.91 (d, J=9.4 Hz, 1 H), 7.72 (d, J=7.3 Hz, 1 H), 7.52 (d, J=8.4 Hz, 1 H), 7.20 (d, J=7.8 Hz, 1 H), 6.68 (s, 3 H), 3.49 (m, 6 H), 3.36 (s, 3 H), 3.21 (s, 6 H), 3.12 (s, 3 H), 3.09 (s, 3 H), 2.71 (s, 3 H), 2.69 (s, 3 H). HRMS (ESI) m/z for C₂H₁₂₃N₁₃NaO₁₅⁺ [M+Na]⁺ calculated: 1432.9154; found: 1432.9165.

### Example 3 Synthesis of compound 8

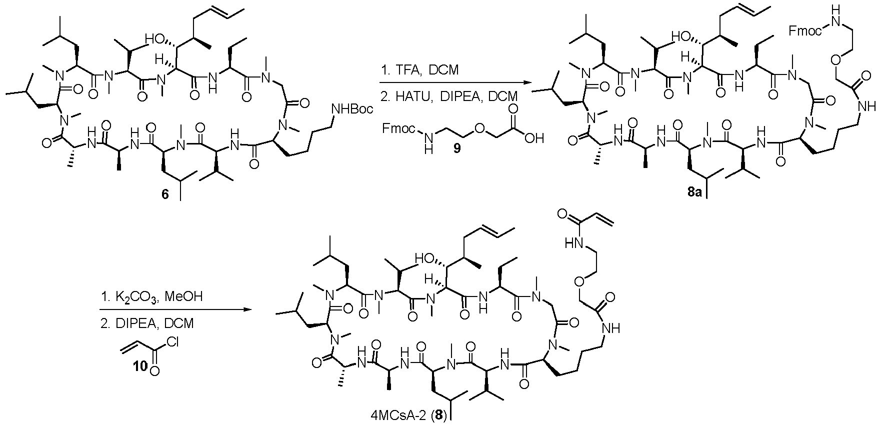

**8a**: Compound **6** was deprotected under TFA conditions to give product (50 mg, 41.06 µmol), **9** (28.03 mg, 82.13 µmol) and HATU (31.23 mg, 82.13 µmol) dissolved in 3 ml DCM, then DIPEA (21.23 mg, 164.25 [µmol, 28.61 µL) was added with stirring and reacted overnight. Product **8a** (50 mg, 32.45 [µmol, 79% yield) as a pale yellow solid is obtained by purification using column chromatography.

**8**: **8a** (12 mg, 7.79 µmol) was dissolved in MeOH (2 mL) and then potassium carbonate (2.15 mg, 15.57 µmol, 0.94 µL) was added and reacted at room temperature to complete the conversion of starting material. After the removal of the solvent by rotary evaporation, the product was directly dissolved in DCM. Then **10** (1.37 mg, 15.17 µmol, 1.25 µL) and DIPEA (1.96 mg, 15.17 µmol, 2.64 µL) were added. After completion of the reaction, product **9** (10.3 mg, 7.4 µmol, 95%) is obtained by extraction and column chromatography.

Characterization data for 4MCsA-2 (**8**): ¹H NMR (500 MHz, CDCl₃) δ 7.97(d, J=9.4 Hz, 1 H), 7.67(d, J=7.3 Hz, 1 H), 7.48(d, J=8.4 Hz, 1 H), 7.15(d, J=7.8 Hz, 1 H). HRMS (ESI) m/z for C₆₉H₁₂₁N₁₃NaO₁₅⁺ [M+Na]⁺ calculated: 1394.8997; found: 1394.9006.

### Example 4 Synthetic route for CsA-FITC probe

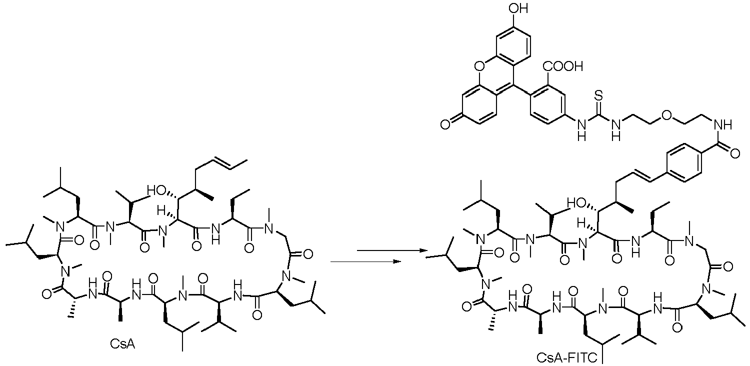

Characterization data for CsA-FITC: HPLC method, gradient (30%-100% ACN, 0-25 min), column temperature 70°C, R = 16.9 min. HRMS (ESI) m/z for C₉₃H₁₃₄N₁₄NaO₁₉S⁺ [M+Na]⁺ calculated: 1805.9563; found: 1805.9536.

### Example 5 Verification of reactivity of 4MCsA with albumin

The reactivity of the molecules with albumin was determined after *in vitro* binding by a Q Exactive HFX mass spectrometer (Thermo Fisher) mass spectrometer using the whole protein method.

Reacting at a ratio of 4MCsA : BSA = 2 : 1 at room temperature *in vitro* for 1 hour, then excess molecules and salts were removed by centrifugation using an ultrafiltration tube, the sample was loaded onto a reversed-phase phenyl column (MAbPac, Thermo Fisher, 088648). The data were collected via LC-ESI-MS, and the entire protein mass was resolved by deconvolution of the original mass spectrum using BioPharma Finder software (Thermo Fisher).

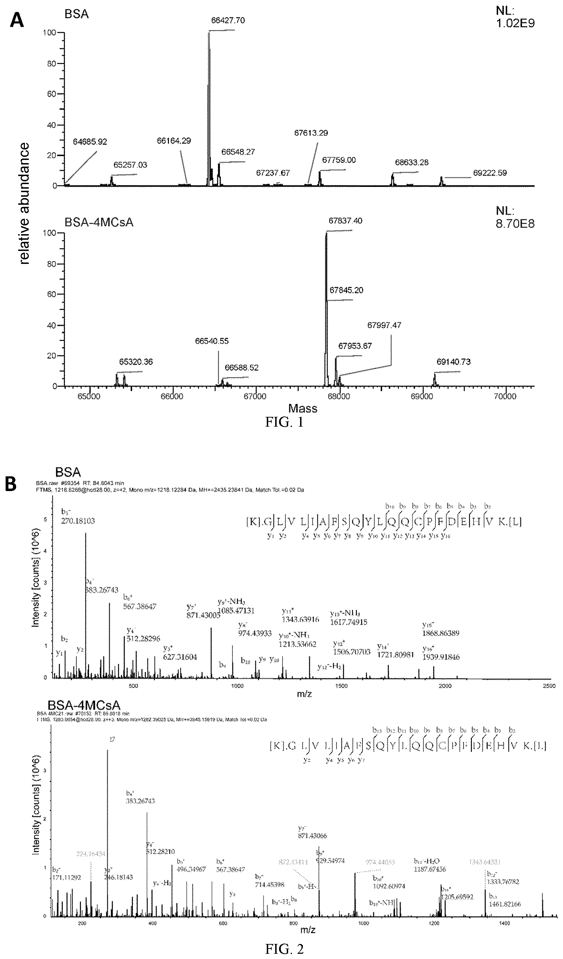

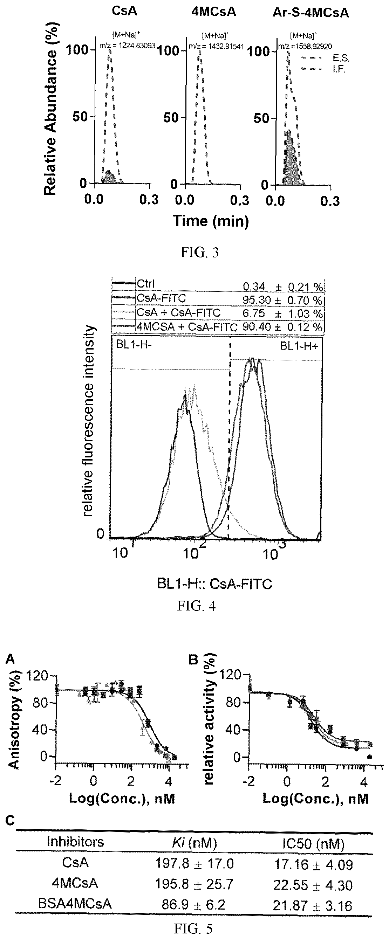

As shown in Fig. 1, 4MCsA increases the molecular weight of albumin by 1410 Da, and 4MCsA can effectively covalently bind albumin at a stoichiometric ratio of 1:1 with good reactivity.

### Example 6 Validation of 4MCsA with albumin cysteine reaction sites

The complex of Example 4 was denatured with urea, and the diluted protein sample was added in the proportion of pancreatin : protein = 1: 50. After enzymatic hydrolysis at 37°C for 18 h, the peptide sample was desalted using Pierce^{®} Spin Columns (Thermo Fisher) desalting column. The eluate was eluted with 70% acetonitrile, lyophilized, dissolved with 0.1% formic acid in water, and the peptide was analyzed by LC-ESI-MS, and the modified sites were analyzed using BioPharma Finder software (Thermo Fisher).

As shown in Fig. 2, the bottom-up peptide identified a peptide fingerprint with the 4MCsA-modified peptide [K] .GLVLIAFSQYLQQcPFDEHVK. [L], showing the 4MCsA molecule on the Cys34 modification. The rapid and specific reactivity of 4MCsA molecules with BSA provides support for their cell non-transmembraneity.

### Example 7 High resolution mass spectrometry determination of molecular penetration in Jurkat cells

For direct detection of molecular penetration, intracellular compound concentrations were quantified by an external standard method using a QE-Focus mass spectrometer.

1×10⁸ Jurkat cells were added to RPMI1640 medium containing 5 µM CsA, 4MCsA, and thiophenol blocked 4MCsA, incubated at 37 °C for 2 hours, then intracellular compound fractions were extracted using methanol for analysis, and extracts were subjected to mass spectrometry using 5 µM methanolic solutions of the corresponding molecules as external standards. The m/z signal of the corresponding molecule is selected to perform peak extraction on the chromatogram to obtain an ion chromatogram (EIC) corresponding to the extraction of the molecule, and then the concentration of the extract is quantified using the peak area under the curve of the EIC of the cell extract. The results are shown in Fig. 3 and Table 1.

**Table 1 Jurkat intracellular compound content**

| Compounds | Abundance ratio (%) | Intracellular conc. (µM) |
|---|---|---|
| CsA | 9.15±0.15 | 7.22±0.12 |
| 4MCsA | NA | NA |
| Ar-S-4MC | 42.51±0.42 | 33.43±0.33 |

Results are shown in Table 1 and Fig. 3. CsA was enriched intracellularly due to its binding to intracellularly abundant CypA, and the concentration of intracellular CsA was higher than extracellular one (7.2 µM *vs.* 5 µM). 4MCsA was not detected intracellularly, whereas hydrophobic thiophenol-blocked 4MCsA was able to enter the cell more easily. After the reaction of the maleimide group in the thiophenol-blocked 4MCsA molecule with thiophenol, it can no longer bound albumin, and its cell membrane permeability is even higher than that of CsA, indicating that the maleimide group in 4MCsA is essential for the cell non-membrane permeability of the molecule. 4MCsA is effectively confined extracellularly.

### Example 8 Determination of membrane permeability of molecules in Jurkat cells by fluorescence competition assay

To further compare the cell permeabilization of CsA and 4MCsA, this example examined the effect of compounds on an intracellular fluorescent signal by flow cytometry using a competition method using a CsA-FITC fluorescent probe that binds to intracellular CypA.

The results are shown in Fig. 4. CsA-FITC was able to efficiently bind intracellular CypA resulting in fluorescence of the cell marker, while CsA entered the cell and competed with the CsA-FITC probe for binding to intracellular CypA, thereby greatly reducing the proportion of fluorescent cells (from 95.30% to 6.75%). In contrast, however, 4MCsA had very little effect on the intracellular fluorescence signal.

In conjunction with the results of Example 6, 4MCsA accomplishes the role of limiting the molecule extracellularly and is an effective molecule for subsequent targeting of extracellular eCypA.

### Example 9 Determination of binding capacity of 4MCsA and complexes with peptidyl-prolyl-cis/trans-isomerase (PPI enzyme)

Fluorescence polarization (FP) is a widely used technique for measuring ligand-receptor binding in solution systems. Free fluorescent probes in the free state rotate rapidly in the system and the emitted fluorescence is mostly depolarized light. Once the probe binds to a larger molecule, the rotation of the molecule is restricted, and the emission of polarized light increases.

The present invention synthesizes a CsA analog labeled with fluorescein isothiocyanate as a fluorescent tracer probe (CsA-FITC) to determine the affinity of ligands for CypA using a competitive fluorescence polarization assay. The binding constant *K_{d}* = 14.81 ± 0.70 nM for CsA-FITC with CypA was first determined. Competition fluorescence polarization curves were further measured using 50 nM CypA in combination with different concentrations of inhibitors followed by the addition of 20 nM CsA-FITC probes.

The results are shown in Fig. 5, with *Kᵢ* of 195.8 ± 25.7 nM for CsA and 197.8 ± 17 nM for 4MCsA with recombinant CypA, respectively. At the same time, the *Kᵢ* of BSA-4MCsA complex with CypA is 86.9 ± 6.2 nM, and the binding capacity of the three complexes with CypA is close, indicating that 4MCsA and the coupling of 4MCsA with BSA do not affect the binding of inhibitor with CypA, and the activity is equivalent to that of CsA.

### Example 10 Inhibition of 4MCsA and complexes on PPI enzyme activity

The effect of inhibitors on CypA enzyme activity was determined using the chymotrypsin hydrolysis of *N*-Succinyl-Ala-Ala-Pro-Phe *p*-nitroanilide substrate peptide. The proline of substrate peptide is mainly cis in the specific component system, while chymotrypsin has low cleavage efficiency. When catalyzed by proline cis-trans isomerase, its configuration changes from cis to trans, which can be specifically cleaved by chymotrypsin and release *p*-nitroanilide with maximum absorption around 390 nm. Thus, the stronger the CypA enzyme activity is, the faster the substrate is degraded, and the faster the absorbance at 390 nm increases. Kinetic monitoring was initiated by the addition of 250 µg/mL chymotrypsin and finally, 80 µM of an ultra-dry substrate peptide solution (470 mM LiCL in trifluoroethanol) using a microplate reader with an autosampler after binding to different concentrations of inhibitors using 50 nM CypA.

As shown in Fig. 5, after incubation of CypA with different concentrations of inhibitor molecules, the enzymatic activity of CypA was significantly inhibited, and the IC50 values of 4MCsA, CsA, and BSA-4MCsA were 22.55 ± 4.30 nM, 17.16 ± 4.09 nM and 21.87 ± 3.16 nM respectively by curve fitting, further indicating that 4MCsA and its conjugate with BSA can effectively inhibit the enzymatic activity of CypA, which is equivalent to the efficiency of CsA, consistent with the determination of binding activity, demonstrating that 4MCsA can normally exert the inhibitory effect of targeting CypA after coupling with BSA.

### Example 11 Detection of non-immunosuppressive effects in NFAT signaling

The immunosuppressive effect of CsAis mainly due to the formation of a CypA/CsA complex with intracellular CypA after the molecule enters the cell and then binds calcineurin, which prevents the activation of NFAT protein dephosphorylation by this enzyme.

Based on this, the present inventors investigated the effect of molecules on NFAT signal transduction during JurkatT cell activation by ionomycin and PMA. After incubation of 1×10⁶ Jurkat cells in RPMI1640 medium (containing 10% serum) supplemented with CsA and 4MCsA for 1.5h, followed by treatment with 25nM phorbol ester PMA and 1 µg/mL ionomycin for 2h, the activation of Jurkat cells was induced. After the collection of cells, the dephosphorylation level of NFAT protein was detected by extraction, electrophoresis, transfer printing, and NFAT antibody. Dephosphorylation of NFAT results in two bands, phosphorylated and nonphosphorylated.

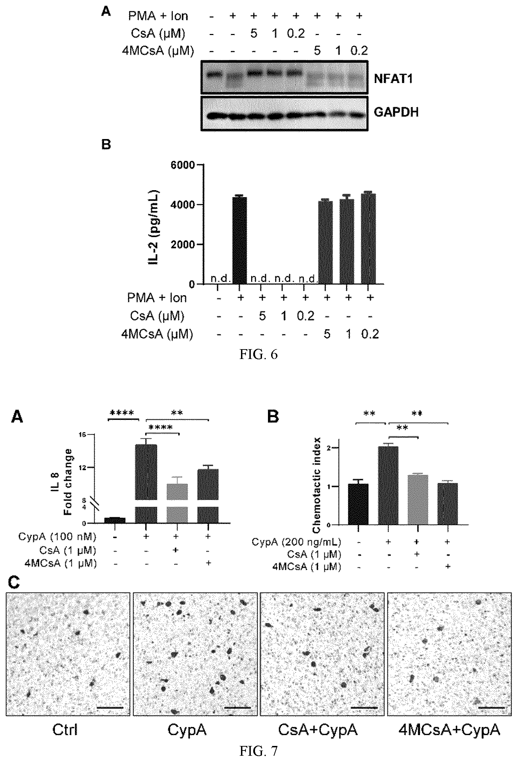

As shown in Fig. 6, CsA significantly inhibited NFAT dephosphorylation induced by PMA and ionomycin, whereas the cell non-penetrating 4MCsA had no significant effect.

Meanwhile, the present invention further examined the secretion of IL-2 protein downstream of T-cell activation in which NFAT was involved, and the IL-2 protein content in the culture medium was measured using the ELISA method after 24h of PMA and ionomycin stimulation. Consistent with the results of the Western blot, 0.2 µMCsA could significantly inhibit the secretion of IL-2, and the concentration of 4MCsA up to 5 µM hardly affected the secretion of IL-2. The above results indicate that 4MCsA has no immunosuppressive effect, which is consistent with its effective confinement outside the cell, and thus its inability to form a complex with intracellular CypA to interfere with calcineurin and NFAT activation.

### Example 12 Effect of 4MCsA on inflammatory cells

The present inventors first demonstrated that 4MCsA promoted the secretion of the pro-inflammatory factor IL-8 by CypA.

Changes in the transcription of CypA-stimulated pro-inflammatory factor IL-8 by 4MCsA and CsA, as well as the migration of inflammatory cells, were examined using CypA proteinstimulated cells after preincubation of drug molecules with culture medium. After pre-incubating 4MCsA and CsA with RPMI1640 medium containing 0.1% BSA for 1 hour, THP1 cells were simultaneously stimulated with CypA for 12 hours, and the transcription level of IL-8 was detected by RT-PCR. In the Transwell migration assay, 4MCsA and CsA were pre-incubated with RPMI1640 medium containing 0.1% BSA for 1 hour in the lower chamber, then 200 ng/mL CypA was added and 1.0×10⁵ THP1 cells were added to the upper chamber and incubated for 1 hour at 37 °C, then the number of migrated cells was photographed after crystal violet staining of the lower chamber membranes.

As shown in Fig. 7, eCypA significantly increased the transcription level of mRNA of IL-8, and this effect was significantly inhibited by 4MCsA and CsA. The present invention then uses Transwell to verify the inhibition of CypA-induced THP-1 cell migration by 4MCsA. The migration activity promoted by CypA was significantly abolished by 4MCsA and CsA. These results indicate that both 4MCsA and CsA can target the CypA pocket, and that 4MCsA can effectively interfere with the inflammatory effects and leukocyte migration mediated by the eCypA pocket.

### Example 13 Comparison of cytotoxicity of 4MCsA with native cyclosporine

This example compares the cytotoxic effects of CsA and non-cell penetrating 4MCsA on normal HEK293 human kidney cells.

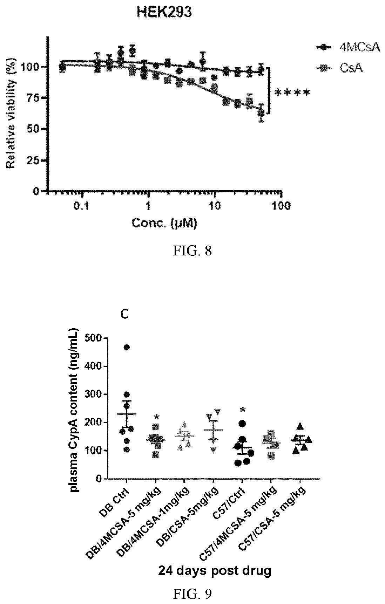

To test the cytotoxicity of the drugs, the MTT method was used to detect the killing effect of cells treated with 4MCsA and CsA. HEK293 cells were seeded into 96-well plates at a density of 1.0×10⁴ well, and the cells were allowed to adhere and grow to an appropriate density; meanwhile, the cells were pre-incubated for 1 hour with the medium containing 4% BSA and gradient concentrations of drugs of 4MCsA and CsA, then the 96-well plate medium was replaced with the drug pre-incubation medium, and the culture was continued for 30 hours, and the cell survival rate was determined by MTT method. As shown in Fig. 8, 4MCsA concentrations reached 50 µM with little cytotoxicity, compared to CsA which significantly inhibited the proliferation of HEK293 cells, showing a concentration-dependent cytotoxic effect with increasing concentrations. This is consistent with the hepatorenal toxic effects of CsA in clinical use, where 4MCsA represents a great advantage.

### Example 14 Administration of 4MCsA reduces CypA in a diabetic model

This example examined the effect of drugs on serum CypA contents in mice using db/db genetically engineered mice deficient in leptin receptors (DB mice) as a T2DM model (C57 mice as a healthy control group). After DB and C57 mice were treated with 5 mg/kg weekly CsA or 4MCsA and 1 mg/kg weekly 4MCsA via tail vein injection for 3 weeks, serum was collected and ELISA was used to detect the content of CypA in blood.

As shown in Fig. 9, blood CypA contents decreased in diabetic mice after dosing, indicating that 4MCsA was able to block the role of extracellular cyclophilins in inflammation and ROS cycle amplification, while reducing CypA secretion during this amplification.

### Example 15 4MCsA reduces fasting blood glucose in a mouse model of T2DM

To effectively evaluate the effect of the drug on T2DM, this example selected DB mice and high fat diet-induced obese mouse models (HFD mice) as two different predisposing mouse models of type 2 diabetes as subjects. DB model was established with control group (Ctrl), 4MCsA 5 mg/kg/week group (DB4MC5), 4MCsA 1 mg/kg/week group (DB 4MC1), and CsA 5 mg/kg/week group (DB CsA5). Both 4MCsA and CsA were administered at 5 mg/kg in the HFD model, and both models were administered once weekly via tail vein injection.

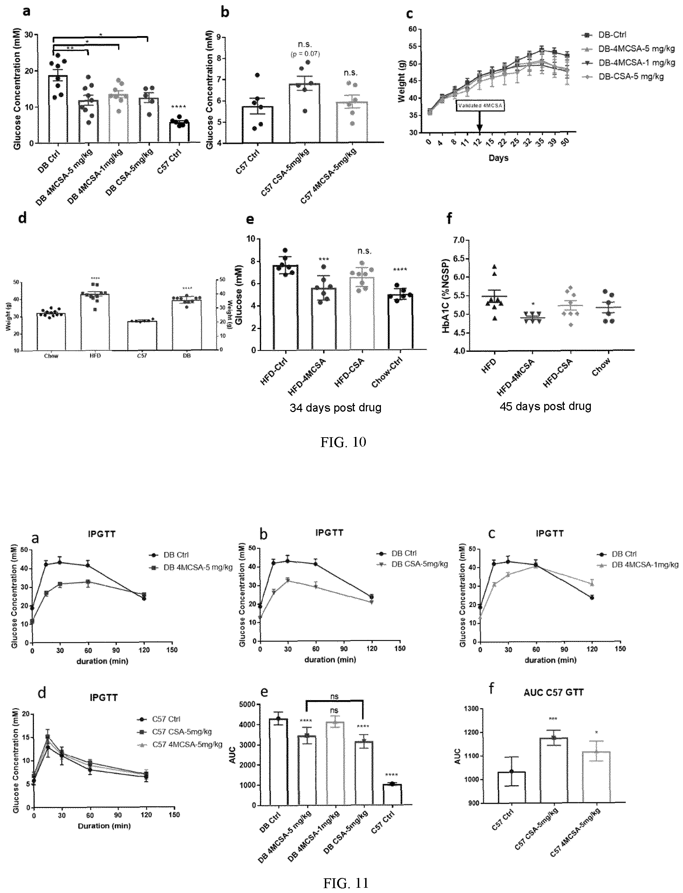

As shown in Fig. 10, 4MCsA significantly decreased fasting blood glucose and decreased glycated hemoglobin contents in DB and HFD mice compared to CsA after administration, indicating that 4MCsA is effective in long-term glycemic control in diabetes. It is also noteworthy that after 5 weeks of dosing on normal C57 mice, fasting blood glucose was slightly higher in the CsA 5 mg/kg/week group than in the C57 ctrl group, while 4MCsA 5 mg/kg/week was comparable to the normal group.

### Example 16 4MCsA improves glucose tolerance in the T2DM mouse model

The glucose tolerance test (GTT) was performed on mice administered with the two models in Example 15 to examine the ability of the mice to control glucose. After 5 weeks of administration, the mice were fasted for 16 hours, followed by intraperitoneal injection of glucose (1 g/kg for DB mice and 2 g/kg for HFD mice), and then blood was collected at different time points to detect the blood glucose content. Blood samples at the corresponding time points were also stored for subsequent glucose-stimulated insulin detection.

As shown in Fig. 11, both 4MCsA and CsA 5 mg/kg significantly improved the glucose tolerance status of DB mice (panels a-b). As shown in panel e, the area under the curve (AUC) was significantly reduced and glucose clearance was faster in vivo in the 5 mg/kg group compared to the Ctrl group. In the GTT curve of the C57 group and its AUC (panels d and f), it can be observed that CsA significantly increased the AUC of C57 healthy mice, while the increase of AUC in the 4MCsA group was less than that in CsA group, indicating that the administration of CsA may have some damage to the glucose tolerance of healthy mice, and the effect of 4MCsA was relatively smaller.

The improvement of GTT in T2DM mice by CsA and 4MCsA was further studied in the HFD model.

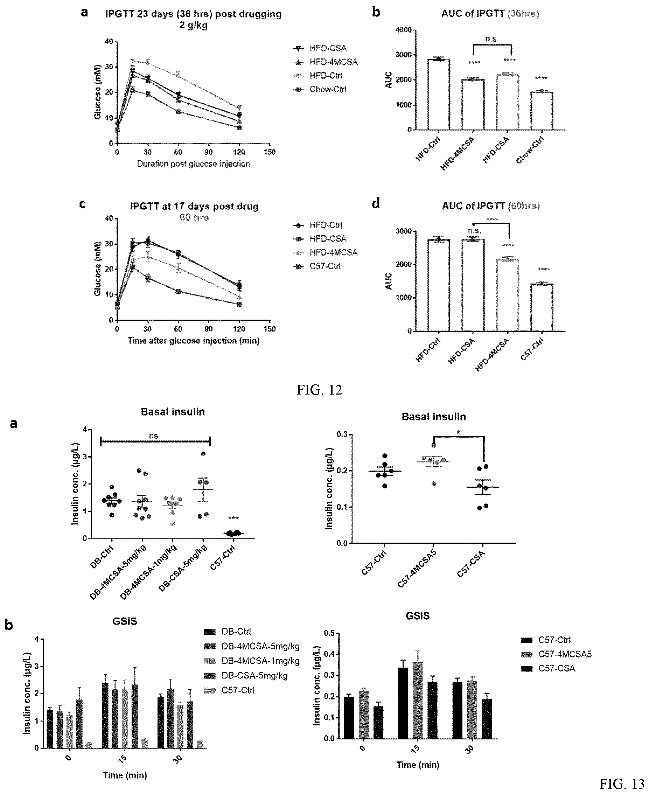

As shown in Fig. 12, in the HFD model, both 4MCsA and CsA showed a significant improvement in GTT of HFD, and a significant decrease in AUC, measured within 36 hours on day 23 of dosing. Within 60 hours on day 17 of dosing, no effect of CsA on GTT of HFD was observed, while 4MCsA remained significantly improved, significantly reducing AUC. The above results show that 4MCsA has a longer metabolic time and can improve inflammation and ROS state for a long time through continuous control of extracellular eCypA, to always exert pharmacological effects.

### Example 17 Effect of 4MCsA on glucose-stimulated insulin secretion

Blood samples corresponding to the time points after glucose injection in Example 16 were taken and the insulin content was determined using ELISA to obtain glucose-stimulated insulin secretion results.

As shown in Fig. 13, the basal level of insulin in DB mice was significantly higher than that in C57 healthy mice, and CsA and 4MCsA had little effect on the basal level of insulin in DB mice after administration (panel a, left), but the insulin in 4MCsA group was slightly higher than that in CsA group in C57 healthy mice (panel a, right). After glucose stimulation, 4MCsA and CsA showed no significant difference in insulin secretion from DB mice stimulated at different stages compared with the DB ctrl group (panel b, left). The use of 4MCsA and CsA 5 mg/kg/day did not change the insulin secretion of DB mice, so the improvement of DB mice GTT was caused by affecting the insulin signaling response. However, in healthy C57 mice (panel b, right), it was still observed that 4MCsA increased glucose-stimulated insulin secretion more than C57 ctrl, while the CsA group decreased glucose-stimulated insulin production, indicating the effect of C57 treated with CsA in the GTT experiment. Thus, the 4MCsA of the present invention was further demonstrated to be superior to CsA.

### Example 18 Effect of 4MCsA on insulin sensitivity

Insulin signaling response was further measured by performing an insulin sensitivity test (insulin tolerance test, ITT) on each of the two mice in Example 15. After the mice were fasted for 6 hours, insulin (2 U/kg for DB mice and 1 U/kg for HFD mice) was injected intraperitoneally, and then the blood glucose contents were measured at different time points.

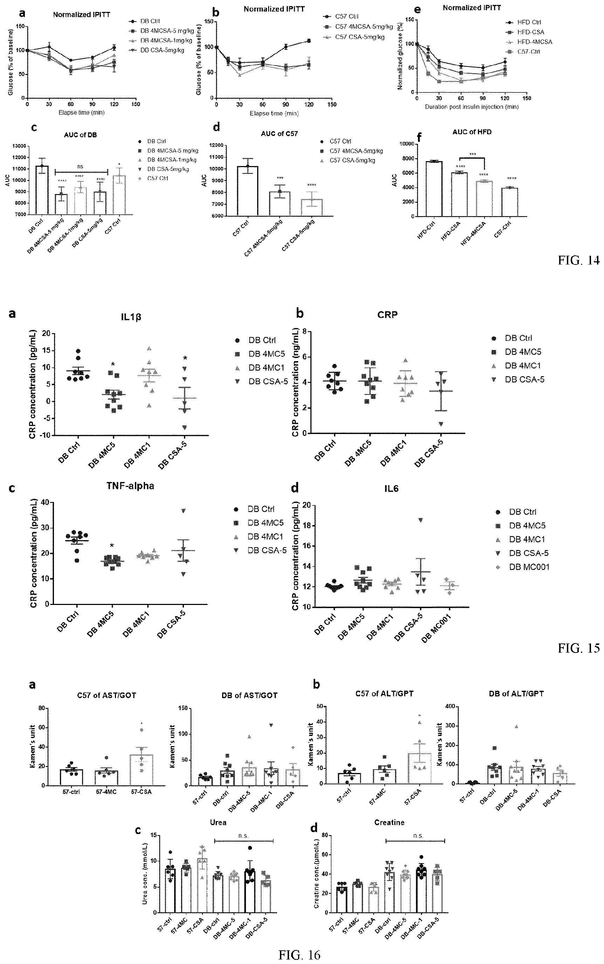

As shown in Fig. 14, both 4MCsA and CsA significantly improved DB insulin sensitivity, while an improvement in ITT was observed in healthy C57 after administration. The effect of 4MCsA on ITT in DB mice was dose-dependent (panels a-d). In mice of the HFD model, 4MCsA and CsA also attenuated insulin resistance caused by obesity, improved ITT, and 4MCsA had lower AUC and a more significant effect than CsA (as shown in panels e-f).

### Example 19 Effect of 4MCsA on DB mouse inflammatory factor

DB model mice in Example 15 (control group (Ctrl), 4MCsA 5 mg/kg/week group (DB 4MC5), 4MCsA 1 mg/kg/week group (DB 4MC1), CsA 5 mg/kg/week group (DB CsA5)) After administration for 42 days, blood was collected and serum inflammatory factor levels were determined by ELISA.

As shown in Fig. 15, 4MCsA could significantly reduce the levels of inflammatory cytokines TNF-α and IL-1β in DB mice after 42 days of administration, while CsA only improved IL-1β. 4MCsA reduces inflammation in mice and has a higher potency than CSA, thereby reducing the impairment of insulin signaling caused by inflammation and improving insulin sensitivity, thereby improving the diabetic state of mice.

### Example 20 Investigation of effect of 4MCsA and CSA on hepatorenal toxicity in DB mice

After 50 days of treatment, the serum of the corresponding treatment groups of the two model mice in Example 15 was taken to detect the contents of ALT, AST (liver toxicity indicators), and nephrotoxic indicators (creatinine and urea) in the serum using biochemical kit (Nanjing Jiancheng Bioengineering Institute).

As shown in a and b in Fig. 16, DB mice had a significant increase in both ALT and AST compared to C57 healthy mice, which may be that obese mice were prone to nonalcoholic fatty liver increasing these liver indices, while hepatorenal toxicity of DB mice was not further enhanced by 4MCsA and CsA (c and d in Fig. 16). Similarly, 4MCsA and CSA did not produce significant nephrotoxicity with no difference in urea and creatinine levels between groups. However, it can still be seen here that in C57 healthy mice, after 50 days of long-term administration, AST, ALT, and urea levels increased significantly in the CsA administration group, while 4MCsA remained close to those of healthy controls. This result more directly demonstrates the advantage of the 4MCsA of the present invention in reducing toxicity.

In both DB mice and HFD T2DM model mice, 4MCsA and CsA were compared in improving T2DM. In disease models, both can reduce fasting blood glucose, improve glucose tolerance, and increase insulin sensitivity without affecting insulin secretion and other changes in hepatorenal toxicity using low-dose long-cycle administration. Among these indicators, 4MCsA showed greater potential, with longer-term continuous control, more obvious improvement effect, earlier onset of action, and lower toxicity than CsA.

It is also noteworthy that since CsA has been reported to be metabolized more rapidly in diabetic subjects, it is potentially harmful to healthy subjects, which has also been demonstrated in experiments of the present invention several times. Long-term administration to healthy C57 mice impairs GTT, reduces insulin secretion, increases blood glucose levels, and increases hepatorenal toxicity. The 4MCsA we designed has a significant reduction in these adverse effects in healthy C57.

### Example 21 4MCsA exerts an anti-tumor effect in a mouse colon cancer tumor model

This example establishes a mouse tumor model to reflect the effect of the drug by measuring the size of the tumor.

CT26-LUC mouse colon cancer cells were inoculated subcutaneously on the dorsal side of Balb/C mice in an amount of 10⁶/mouse. Six days later, the tumor size was about 100 mm³. The simultaneous administration was measured with vernier caliper and the administration dose of 4MCsA was 5 mg/kg. The tumors were measured once every two days and the tumor size was counted. The experiment was terminated after three weeks.

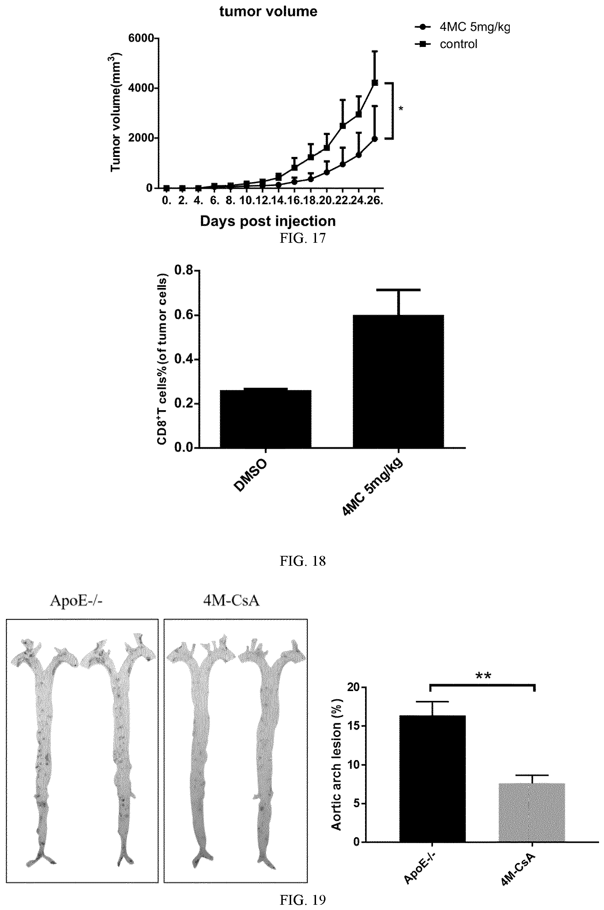

Experimental results are shown in Fig. 17. 4MCsA showed a certain antitumor effect and had potential as an antitumor drug.

### Example 22 Investigation of 4MCsA enhances CD8T cell infiltration in the tumor microenvironment

The thickness of the frozen section is required to be 4-8 µm, place it at room temperature for 30 min after taking it out from -80°C, fix it in acetone at 4°C for 10 min, wash with PBS, 5 min×3; block with 5-10% normal goat serum (diluted in PBS) and incubate at room temperature for 10 minutes. Pour out the serum, do not wash, drop the primary antibody or primary antibody working solution diluted in appropriate proportion, and incubate at 37°C for 1-2 hours or at 4°C overnight. PBS flush, 5 min×3 times; add the appropriately diluted biotin-labeled secondary antibody (diluted in 1% BSA-PBS) dropwise, and incubate at 37°C for 90-120 minutes; or second-generation horseradish enzyme-labeled streptavidin working solution, incubate at 37°C or room temperature for 10-30 minutes; the PBS was washed 5 minutes×3 times, mounted and photographed using an inverted fluorescence microscope. Finally, ImageJ was used for statistical analysis.

Results are shown in Fig. 18, 4MCsA was able to promote the infiltration of CD8T cells in the tumor microenvironment, thereby achieving tumor suppression.

### Example 23 Effect of 4MCsA on aortic lesion extent in high fat diet induced atherosclerotic mice

Five-week-old ApoE gene-deficient C57BL/6J mice were randomly divided into two groups and fed with the Western diet for high-fat induction. From the 7th week, the experimental group received 4MCsA at a dose of 5 mg/kg/week via tail vein injection for 9 weeks. At the end of the experiment, the experimental animals were euthanized, dissected, and perfused with 10 mL of tissue fixative (4% paraformaldehyde) and 10 mL of mL PBS from the right atrial appendage. The aorta was isolated intact under stereoscope and extravascular adherent adipose tissue was dissected with fat forceps. The isolated aorta was stained with oil red O, photographed, and recorded, and the lesion area of the aortic arch in the experimental group and the control group was counted with ImageJ, and the difference was statistically significant.

As shown in Fig. 19, the lesion area of the aortic arch was significantly reduced in the treatment group (4MCsA) relative to the control group (ApoE^{-/-}) after periodic intermittent administration in atherosclerosis mice successfully modeled, indicating that 4MCsA can directly improve the occurrence of vascular plaque lesions.

### Example 24 Effect of 4MCsA on serum CRP content in high-fat diet-induced atherosclerotic mice

After successful modeling of atherosclerotic mice, the effect of the compounds on the development of cardiovascular disease was evaluated by assessing the risk of developing cardiovascular disease in the body by measuring CRP levels in the serum of experimental mice given weekly tail vein injections of 5 mg/kg 4MCsA for 9 weeks as described in Example 23.

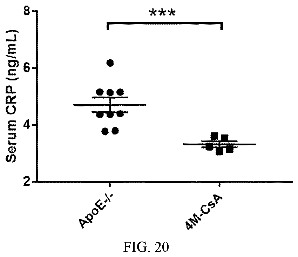

As shown in Fig. 20, compared with the control group (ApoE^{-/-}), 4MCsA could very significantly reduce the CRP content in the serum of mice in the experimental group, indicating that 4MCsA has a very good predictive improvement effect on the occurrence of cardiovascular disease in the body.

### Example 25 Effect of 4MCsA on conventional blood lipid markers in serum of high-fat diet-induced atherosclerotic mice

A mouse model of atherosclerosis induced by a high-fat diet was established as described in Example 23 and administered in groups, 4MCsA at 5 mg/kg or placebo via tail vein once a week for 9 consecutive weeks, after which the mice were euthanized and serum was collected for routine lipid profile testing.

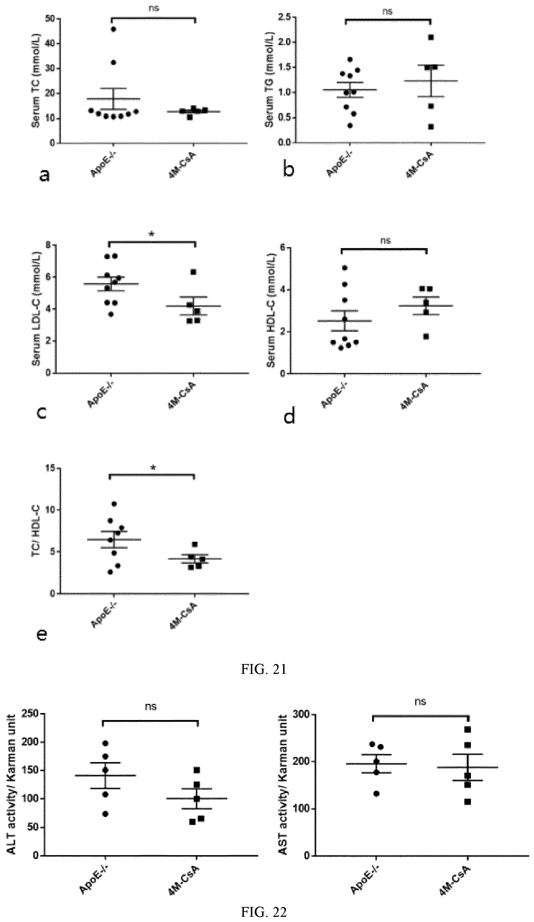

Results are shown in Fig. 21. Compared with the control group, 4MCsA significantly decreased the serum LDL-C content, and increased the HDL-C content to a certain extent, but had no significant effect on the TC and TG content; in addition, the results show that 4MCsA can significantly reduce TC/HDL-C, and thus have a certain role in predicting and improving cardiovascular diseases such as atherosclerosis and coronary heart disease.

### Example 26 Effect of chronic injection of 4MCsA on serum ALT, AST contents in high-fat diet-induced atherosclerotic mice

Atherosclerosis model in mice was induced and administered as described in Example 25. At the end of the experimental period, the mice were euthanized and serum was taken for determination of ALT and AST contents.

As shown in Fig. 22, there was no significant change in the activity of ALT and AST in the serum of the experimental group compared with the control group, indicating that the periodic intermittent tail vein injection of 4MCsA did not cause significant hepatotoxicity to the body.

Although the preferred embodiments of the present invention have been disclosed for illustrative purposes, those skilled in the art will appreciate that various modifications, additions, and substitutions are possible, without departing from the scope and spirit of the present invention as disclosed in the accompanying claims. Accordingly, the protection scope of the present invention shall be subject to the claims.

## Claims

1. An extracellular cyclophilin inhibitor, **characterized by** having a structure shown in Formula (I): 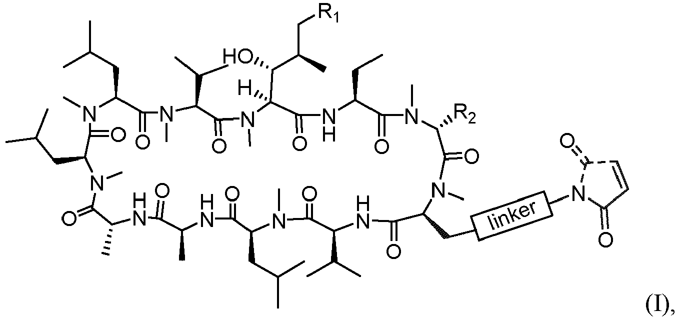 wherein,
R₁ is -CH=CHR₁' or -CH₂CH₂R₁'; wherein, R₁' is selected from alkyl, carboxyl, acetamino, or phenyl;
R₂ is H, SR₂', CH₂SR₂', or CH₂OR₂'; wherein, R₂' is selected from alkyl, carboxyl, hydroxy, acetamino, or phenyl; and
the linker is selected from -(CH₂)*ₓ*-NH(C=O)-(CH₂)*_{y}*- or -(CH₂)*ₓ*-NH(C=O)-(CH₂CH₂O)*_{y}*-; wherein, *x* is an integer of 1-4, and *y* is an integer of 1-6.

2. The extracellular cyclophilin inhibitor according to claim 1, **characterized in that**, R₁' is selected from -CH₃, -(CH₂)*ₙ*-COOH, -(CH₂)*ₙ*-NH(C=O)CH₃, -phenyl, or cycloalkyl; wherein, n is an integer of 1-6.

3. The extracellular cyclophilin inhibitor according to claim 2, **characterized in that**, the phenyl is substituted, one or more, identically or differently, by the following groups: -COOCH₃ or -CH₂NH(C=O)CH₃.

4. The extracellular cyclophilin inhibitor according to claim 2 or 3, **characterized in that**, the cycloalkyl is cyclopropyl.

5. The extracellular cyclophilin inhibitor according to claim 1, **characterized in that**, R₂' is selected from -CH₃, -(CH₂)*ₙ*-COOH, -(CH₂)*ₙ*-OH, -(CH₂)*ₙ*-NH(C=O)CH₃, -phenyl, or cycloalkyl; wherein, n is an integer of 1-6.

6. The extracellular cyclophilin inhibitor according to claim 5, **characterized in that**, the phenyl is substituted, one or more, identically or differently, by the following groups: -COOCH₃ or -CH₂NH(C=O)CH₃.

7. The extracellular cyclophilin inhibitor according to claim 1, **characterized in that**, R₁ is - CH=CHCH₃, R₂ is H, and the linker is selected from -(CH₂)*ₓ*-NH(C=O)-(CH₂)*_{y}*- or -(CH₂)*ₓ-*NH(C=O)-(CH₂CH₂O)*_{y}*-; wherein, *x* is an integer of 1-4, and *y* is an integer of 1-6.

8. The extracellular cyclophilin inhibitor according to claim 1 or 7, **characterized in that**, R₁ is -CH=CHCH₃, R₂ is H, and the linker is -(CH₂)*ₓ*-NH(C=O)-(CH₂)*_{y}*-; wherein, *x* is 2 or 3, and *y* is 3, 4, or 5.

9. A pharmaceutical composition of the extracellular cyclophilin inhibitor according to any one of claims 1 to 8 and a non-toxic pharmaceutically acceptable salt thereof as active ingredients.

10. Use of the extracellular cyclophilin inhibitor according to any one of claims 1 to 8 in the manufacture of a medicament for prevention or treatment of cyclophilin-mediated diseases.
